(19) Europäisches Patentamt
European Patent Office
Office européen des brevets

(11) **EP 3 424 467 A1**

(12) **EUROPEAN PATENT APPLICATION**
published in accordance with Art. 153(4) EPC

(43) Date of publication:
**09.01.2019 Bulletin 2019/02**

(51) Int Cl.:
***A61F 2/44*** (2006.01)   ***A61L 27/00*** (2006.01)

(21) Application number: **17760000.4**

(86) International application number:
**PCT/JP2017/007798**

(22) Date of filing: **28.02.2017**

(87) International publication number:
**WO 2017/150534 (08.09.2017 Gazette 2017/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.02.2016 JP 2016038135**

(71) Applicant: **Medtronic Sofamor Danek, Co., Ltd. Osaka-shi, Osaka 553-0003 (JP)**

(72) Inventors:
• **SUGINO, Atsushi**
  **Osaka-shi**
  **Osaka 553-0003 (JP)**
• **FUKUZAKI, Satoshi**
  **Tsu-shi**
  **Mie 514-8507 (JP)**

(74) Representative: **FRKelly**
  **27 Clyde Road**
  **Dublin D04 F838 (IE)**

(54) **ANTIBACTERIAL APPARATUS FOR IN-VIVO IMPLANTATION**

(57) An antimicrobial interbody cage made of a first material and a second material, wherein a body of the interbody cage comprises the second material; at least a part of the body of the interbody cage is coated with the first material; at least a surface of the first material comprises an antimicrobial metal; at least a surface of the second material is nobler than the first material; an electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material.

# Fig. 1

EP 3 424 467 A1

**Description**

TECHNICAL FIELD

The present invention relates to an implant device, and specifically relates to an implant device that achieves an antimicrobial property while it is implanted in vivo for use.

BACKGROUND ART

A variety of instruments such as cages, wires, plates, screws, rods, connectors, crosslinks, hooks, set screws, artificial vertebral bodies, and artificial intervertebral discs are used as orthopedic implant devices. These instruments may be contaminated with microorganisms when they are implanted in vivo for use. If an implant device is colonized by microorganisms, it is very difficult to prevent microbial growth. To prevent microbial growth and infection in the body, it is necessary to perform surgery to remove the implant device for replacement with a new one, which places an enormous burden on the patient. It is desired that an antimicrobial implant device be provided.

As a technique for imparting an antimicrobial property to an implant device, a medical implant system has been proposed (Patent Literature 1) which comprises a metal component containing an antimicrobial metal disposed on an external surface of the implant body; a power source having a first terminal and a second terminal, one of the terminals being in electrical communication with the metal component; and an insulator placed in a current path between the first terminal of the power source and the second terminal of the power source, preventing current flowing from the first terminal from reaching the second terminal without completing a circuit including a conductive body tissue or fluid adjacent to the external surface of the implant system when implanted. Patent Literature 1 discloses that examples of the antimicrobial metal include silver, copper, both silver and copper, both silver and cadmium, and a combination of silver, copper, and cadmium. In Patent Literature 1, the external power source equipment and the terminals are required in addition to the implant body to achieve an antimicrobial property, and a treatment or an external operation is needed to impart an antimicrobial property. Further improvement is required from the viewpoint of reducing the burden on patients.

CITATION LIST

PATENT LITERATURE

PTL 1: Japanese Patent No. 5175185

SUMMARY OF INVENTION

TECHNICAL PROBLEM

It is an object of the present invention to provide an implant device that can achieve an antimicrobial property, simply by being implanted in vivo, to reduce the burden on patients.

SOLUTION TO PROBLEM

Specific embodiments of the present invention are as set forth below:

[1] An antimicrobial implant device made of a first material and a second material, wherein
at least a surface of the first material comprises an antimicrobial metal;
at least a surface of the second material is nobler than the first material;
an electric circuit is formed between the first material and the second material when the device is implanted in vivo for use; and
ions of the antimicrobial metal are eluted from the first material under a presence of an electrolyte to impart an antimicrobial property to a surface and/or a surrounding area of the first material.
[2] The antimicrobial implant device according to [1], wherein the first material comprises at least one antimicrobial metal selected from cobalt, silver, zinc, copper, tungsten, magnesium, nickel and phosphorus.
[3] The antimicrobial implant device according to [1] or [2], wherein the first material is a cobalt-based alloy.
[4] The antimicrobial implant device according to [3], wherein the cobalt-based alloy comprises cobalt in an amount of 29 to 69 mass%.
[5] The antimicrobial implant device according to [3] or [4], wherein the cobalt-based alloy is a cobalt-chromium alloy.
[6] The antimicrobial implant device according to [5], wherein the cobalt-chromium alloy comprises chromium in an amount of 18 to 30 mass%.

[7] The antimicrobial implant device according to any one of [1] to [6], wherein the second material is titanium or a titanium-based alloy.

[8] The antimicrobial implant device according to [7], wherein the titanium or the titanium-based alloy comprises titanium in an amount of 68 to 100 mass%.

[9] The antimicrobial implant device according to any one of [1] to [8], wherein a ratio of a surface area of the second material to a surface area of the first material (second material/first material) is in a range of 0.2 to 10.8.

[10] The antimicrobial implant device according to any one of [2] to [9], wherein cobalt ions are eluted from the first material in an amount of 7 nmol/l to 81 $\mu$mol/l.

[11] The antimicrobial implant device according to any one of [1] to [10], wherein

the antimicrobial implant device is an interbody cage;

a body of the interbody cage comprises the second material; and at least a part of the body of the interbody cage is coated with the first material.

[12] The antimicrobial implant device according to [11], wherein

at least a part of the body of the interbody cage has a honeycomb structure or a net structure composed of the second material; and

the outer surface of the honeycomb structure or the net structure is coated with the first material.

[13] The antimicrobial implant device according to any one of [1] to [10], wherein

the antimicrobial implant device is an interbody cage; and

a porous structure composed of intertwined or agglutinated fibers comprising the first material is laminated on a porous structure composed of intertwined or agglutinated fibers comprising the second material.

[14] The antimicrobial implant device according to any one of [1] to [12], wherein

the antimicrobial implant device is an interbody cage;

a body of the interbody cage comprises a solid portion comprising the second material and a porous section comprising the second material; and

the solid portion and the porous portion are laminated.

[15] The antimicrobial implant device according to any one of [1] to [12], wherein

the antimicrobial implant device is an interbody cage;

a body of the interbody cage comprises the second material; and

a porous portion comprising the first material is laminated on the porous portion comprising the second material.


ADVANTAGEOUS EFFECTS OF INVENTION

The implant device of the present invention, once implanted in vivo, can achieve an antimicrobial property without requiring an external operation or treatment, thereby significantly reducing the burden on patients.

BRIEF DESCRIPTION OF DRAWINGS

Fig. 1 is a perspective view of an interbody cage of the present invention.

Fig. 2 is a cross-sectional view of Fig. 1.

Fig. 3 is a perspective view of a modification of the interbody cage whose inside has a honeycomb structure.

Fig. 4 is a partially enlarged view of the honeycomb structure shown in Fig. 3.

Fig. 5 is a perspective view of a modification in which a body of the interbody cage has a net structure.

Fig. 6 is a perspective view of a modification in which a body and a coating of the interbody cage are made of a porous structure of fibers.

Fig. 7 is a cross-sectional view of Fig. 6.

Fig. 8 is a cross-sectional view of a modification in which a porous portion formed by sintering a plurality of powder particles made of a first material is laminated on a solid portion formed by melting a plurality of powder particles made of a second material to form a coating.

Fig. 9 is an explanatory diagram showing the laminated state of the solid portion and the porous portion.

Fig. 10 is a cross-sectional view of a modification in which a porous portion made of the first material is laminated on a porous portion made of the second material.

Fig. 11 is an explanatory diagram showing the laminated state of the porous portion and the porous portion.

DESCRIPTION OF EMBODIMENTS

The present invention will be described with reference to the attached drawings.

An antimicrobial implant device of the present invention is an implant device made of a first material and a second material, wherein at least a surface of the first material comprises an antimicrobial metal; at least a surface of the second material is nobler than the first material; an electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material. The antimicrobial implant device of the present invention is made of at least two materials, and simply because these two materials are contacted in vivo, a short circuit occurs to form an electric circuit.

Preferably, the first material comprises at least one antimicrobial metal selected from cobalt, silver, zinc, copper, tungsten, magnesium, nickel and phosphorus, and is preferably a cobalt-based alloy. More preferably, the cobalt-based alloy comprises cobalt in an amount of 29 to 69 mass%. Still more preferably, the cobalt-based alloy is a cobalt-chromium alloy comprising chromium in an amount of 18 to 30 mass%.

Preferably, the second material is titanium or a titanium-based alloy. More preferably, the titanium or the titanium-based alloy comprises titanium in an amount of 68 to 100 mass%.

More preferably, a ratio of a surface area of the second material to a surface area of the first material (second material/first material) = 0.2 to 10.8.

When the first material made of the cobalt-based alloy comprising cobalt in an amount of 29 to 69 mass% and the second material made of the titanium-based alloy comprising titanium in an amount of 68 to 100 mass% are used in combination, cobalt ions are eluted from the first material in an amount of 7 nmol/l to 81 $\mu$mol/l to provide a good antimicrobial property.

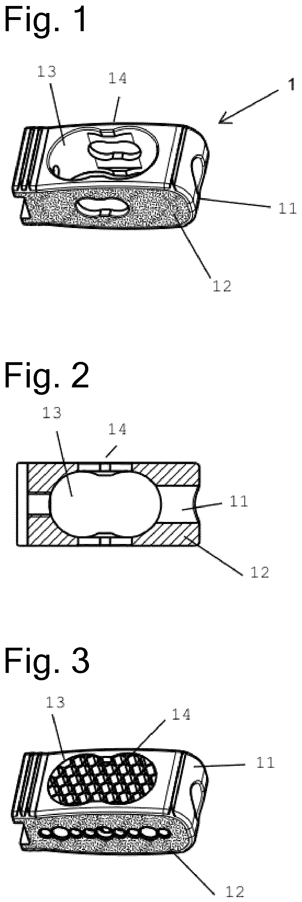

Fig. 1 is a perspective view of an interbody cage 1, and Fig. 2 is a cross-sectional view of Fig. 1. The interbody cage filled with bone grafts is implanted in the intervertebral disc space for use. The interbody cage 1 comprises a body 11 having an opening 13 for charging bone grafts and an inside hollow portion, and a coating 12 that coats at least a surface of the body. The interbody cage 1 can also adopt components of a general interbody cage, as required, as other components. Wall surfaces forming the body 11 of the interbody cage 1 are made of the second material, and at least the surface of the body 11 is coated with the first material. At least the surface of the first material comprises an antimicrobial metal, at least the surface of the second material is nobler than the first material, and the first material and the second material are a combination of materials having a potential difference, such that the contact between the body 11 and the coating 12 causes a short circuit to form an electric circuit, and antimicrobial metal ions are eluted from the first material of the coating 12 under the presence of an electrolyte to impart an antimicrobial property to the surface and/or a surrounding area of the interbody cage body 11.

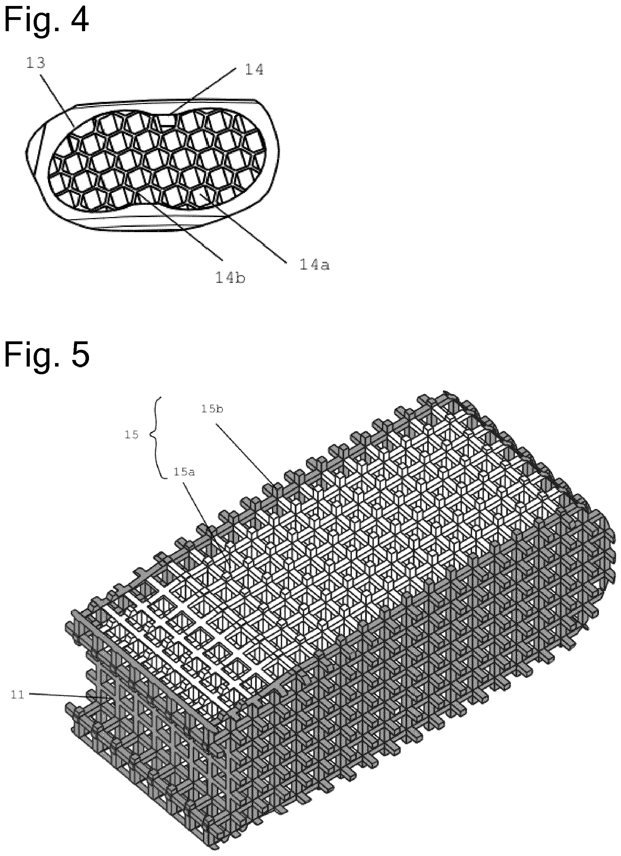

Fig. 3 is a perspective view of a modification in which the opening 13 and the inside hollow portion of the body 11 of the interbody cage 1 are replaced with a honeycomb structure 14, and Fig. 4 is a partially enlarged view of the honeycomb structure 14 shown in Fig. 3. Each wall 14a of the honeycomb structure is made of the second material, and the first material is provided in the coating 12. Because the honeycomb structure in which the inside of the body 11 of the interbody cage 1 is made of the second material is adopted, the surface area of the second material can be increased to increase the ratio of the surface area of the second material to the surface area of the first material, resulting in an increased amount of antibacterial metal ions eluted from the first material.

Fig. 5 is a perspective view of a modification in which the body 11 of the interbody cage 1 has a net structure 15. A frame portion 15a of the net structure 15 is made of the second material, and at least a surface 15b of the net structure 15 that forms an outer surface of the interbody cage body 11 is coated with the first material. The net structure of the interbody cage body 11 allows bone grafts to be loaded from any place, which eliminates the necessity for an opening provided in a general interbody cage. Because the net structure made of the second material is adopted for the body 11 of the interbody cage 1, the surface area of the second material can be increased to increase the ratio of the surface area of the second material to the surface area of the first material, resulting in an increased amount of antibacterial metal ions eluted from the first material.

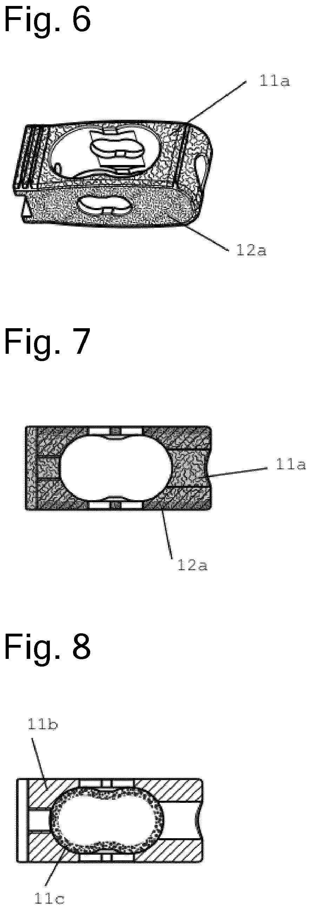

Fig. 6 is a perspective view of a modification in which the body 11 of the interbody cage 1 is made of a porous structure 11a composed of intertwined or agglutinated fibers comprising the second material, and a porous structure 12a composed of intertwined or agglutinated fibers comprising the first material is laminated on the porous structure 11a to form the coating 12. Fig. 7 is a cross-sectional view of Fig. 6. Because both the first material and the second material have a fibrous shape, the surface areas of both materials increase to increase the contact area between the first material and the second material. Furthermore, because the porous structures are adopted, the amount of antibacterial metal ions eluted can be increased, and the elution of the ions can be promoted.

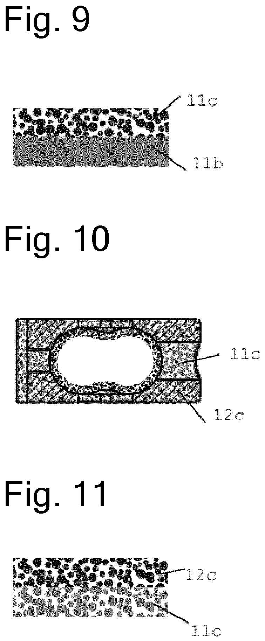

Fig. 8 is a top view of a modification in which the body 11 of the interbody cage 1 is made of a solid portion 11b formed by melting powder particles of the second material, a porous portion 11c formed by sintering powder particles of the second material is laminated on the solid portion 11b, and the porous portion 11c is coated with the first material to have the coating 12 thereon. Fig. 9 is an explanatory diagram showing the laminated state of the solid portion 11b and the porous portion 11c. While the porous portion 11c is laminated on the solid portion 11b in the illustrated embodiment, the solid portion 11b may be laminated on the porous portion 11c. To laminate the solid portion and the porous portion, compression molding, sintering, diffusion bonding, additive manufacturing using an electron beam or laser, metal injection molding, spark plasma sintering, or a combination of these methods can be suitably used. The presence of the porous portions containing the second material as powder particles can increase the surface area of the second material to increase the ratio of the surface area of the second material to the surface area of the first material, resulting in an increased amount of antibacterial metal ions eluted from the first material.

Fig. 10 is a top view of a modification in which the body 11 of the interbody cage 1 is made of a porous portion 11c formed by sintering powder particles of the second material, and a porous portion 12c formed by sintering powder particles of the first material is laminated on the porous portion 11c to form the coating. Fig. 11 is an explanatory diagram showing the laminated state of the porous portion 11c and the porous portion 12c. To laminate the porous portion 11c and the porous portion 12c, compression molding, sintering, diffusion bonding, additive manufacturing using an electron beam or laser, metal injection molding, spark plasma sintering, or a combination of these methods can be suitably used. The presence of the porous portions of the first material and the second material can increase the surface areas of both materials to increase the contact area between the first material and the second material, resulting in an increased amount of antibacterial metal ions eluted from the first material.

EXAMPLES

The present invention will be hereinafter described in detail with examples; however, the invention is not limited to these examples.

[Example 1]

(Preparation of Samples)

A commercially available biocompatible titanium alloy (titanium content: 88 to 91 mass%) and a commercially available biocompatible cobalt-chromium alloy (cobalt content: 58 to 69 mass%, chromium content: 26 to 30 mass%) were processed into 3-mm-thick coin-shaped samples (only the titanium alloy of Sample No. 3 was processed into a flat plate-shaped sample) having the various areas shown in Table 1, and these samples were laminated in the various combinations shown in Table 1 and shorted to each other. As comparative samples, a 1-mm-thick flat plate-shaped polyethylene sample (control), a 3-mm-thick coin-shaped sample made of the cobalt-chromium alloy (Comparison 1), and a 3-mm-thick coin-shaped sample made of the titanium alloy (Comparison 2) were similarly prepared.

[Table 1]

Table 1 Sample Conditions

| Sample No. | Area Ratio* | Surface Area/mm$^2$ | |
| --- | --- | --- | --- |
| | | Titanium Alloy | Cobalt-Chromium Alloy |
| 1 | 1.0 | 518 | 518 |
| 2 | 3.3 | 1696 | 518 |
| 3 | 10.8 | 5600 | 518 |
| 4 | 0.2 | 103 | 518 |
| Comparison 1 | - | - | 518 |
| Comparison 2 | - | 518 | - |
| Control | - | Polyethylene | |
| * Area Ratio = Surface Area of Titanium Alloy/Surface Area of Cobalt-Chromium Alloy | | | |

(Antimicrobial Property Test)

An antimicrobial property was evaluated with reference to JIS Z 2801: Antibacterial products - Test for antibacterial activity and efficacy.

Each of the samples shown in Table 1 was placed in a petri dish, and 40 μl of *Staphylococcus aureus* (NBRC 12732) suspended in a nutrient broth (1/100) was added dropwise. A polyethylene film was placed over the test bacterial suspension added. The suspension was cultured at 35°C for 24 hours, and then cells were washed out from the sample. The resulting wash-out suspension was serially diluted in a 10-fold dilution series and cultured at 35°C for 24 hours using the agar plate culture method, and the number of viable cells was calculated from the number of colony forming units (CFUs) formed. The difference between the logarithmic value of the number of viable cells on the polyethylene sample as a control after 24-hour contact (Nc) and the logarithmic value of the number of viable cells on each sample (N) was determined as the antimicrobial activity value.
[Math. 1]

$$\text{Antimicrobial activity value} = \log(Nc/N)$$

Table 2 shows the number of viable cells and the antimicrobial activity value for each sample. A sample having an antimicrobial activity value of 2.0 or more was determined to have antimicrobial efficacy. The number of viable cells decreased (the antimicrobial activity value increased) as the area ratio (the surface area of the sample made of the titanium alloy/the surface area of the sample made of the cobalt-chromium alloy) increased.

[Table 2]

Table 2 Results of Antimicrobial Property Test

| Sample | Number of Viable Cells/CFU/Coupon | Antimicrobial Activity Value |
|---|---|---|
| Control: Polyethylene | $1.1 \times 10^6$ | - |
| 1 | $6.8 \times 10^3$ | 2.21 |
| 2 | $2.4 \times 10^3$ | 2.66 |
| 3 | $4.0 \times 10^2$ | 3.44 |
| Comparison 1 | $9.2 \times 10^3$ | 2.08 |

[Example 2]

(Preparation of Samples)

An implant device made of the titanium alloy and the cobalt-chromium alloy (the area ratio of the titanium alloy to the cobalt-chromium alloy was set to 0.89) was prepared (implant device 1). As a comparative example, an implant device made of only the titanium alloy having completely the same shape was similarly prepared (implant device 2).

[Table 3]

Table 3 Composition of Implant Device

| Implant Device No. | Area Ratio* | Combination of Materials | |
|---|---|---|---|
| | | Titanium Alloy | Cobalt-Chromium Alloy |
| 1 | 0.89 | ○ | ○ |

(continued)

| Implant Device No. | Area Ratio* | Combination of Materials | |
| --- | --- | --- | --- |
| | | Titanium Alloy | Cobalt-Chromium Alloy |
| 2 | --- | ○ | |
| * Area Ratio = Surface Area of Member Made of Titanium Alloy/Surface Area of Member Made of Cobalt-Chromium Alloy | | | |

(Antimicrobial Property Test)

Each of the implant devices was placed in a polypropylene tube, and immersed in 1 ml of *Staphylococcus aureus* (NBRC 12732) suspended in a nutrient broth (1/100). After 24 hours of culture at 35°C, the culture medium was collected. The collected culture medium was serially diluted in a 10-fold dilution series and cultured at 35°C for 24 hours using the agar plate culture method, and the number of viable cells was determined from the number of colony forming units (CFUs) formed.

Table 4 shows the number of viable cells and the antimicrobial activity value for each implant device. In the implant device 1 in which the area ratio of the titanium alloy to the cobalt-chromium alloy was appropriately set, the number of viable cells significantly decreased. In contrast, in the implant device 2 made of the titanium alloy only, a decrease in the number of viable cells was not observed.

[Table 4]

Table 4 Results of Antimicrobial Property Test for Implant Device

| Implant Device No. | Number of Viable Cells/CFU/Coupon | Antimicrobial Activity Value |
| --- | --- | --- |
| Polyethylene | $7.6 \times 10^7$ | - |
| 1 | $1.2 \times 10^5$ | 2.80 |
| 2 | $7.0 \times 10^7$ | 0.04 |

Claims

1. An antimicrobial implant device made of a first material and a second material, wherein
   at least a surface of the first material comprises an antimicrobial metal;
   at least a surface of the second material is nobler than the first material;
   an electric circuit is formed between the first material and the second material when the device is implanted in vivo for use; and
   ions of the antimicrobial metal are eluted from the first material under a presence of an electrolyte to impart an antimicrobial property to a surface and/or a surrounding area of the first material.

2. The antimicrobial implant device according to claim 1, wherein the first material comprises at least one antimicrobial metal selected from cobalt, silver, zinc, copper, tungsten, magnesium, nickel and phosphorus.

3. The antimicrobial implant device according to claim 1 or 2, wherein the first material is a cobalt-based alloy.

4. The antimicrobial implant device according to claim 3, wherein the cobalt-based alloy comprises cobalt in an amount of 29 to 69 mass%.

5. The antimicrobial implant device according to claim 3 or 4, wherein the cobalt-based alloy is a cobalt-chromium alloy.

6. The antimicrobial implant device according to claim 5, wherein the cobalt-chromium alloy comprises chromium in an amount of 18 to 30 mass%.

7. The antimicrobial implant device according to any one of claims 1 to 6, wherein the second material is titanium or a titanium-based alloy.

8. The antimicrobial implant device according to claim 7, wherein the titanium or the titanium-based alloy comprises titanium in an amount of 68 to 100 mass%.

9. The antimicrobial implant device according to any one of claims 1 to 8, wherein a ratio of a surface area of the second material to a surface area of the first material (second material/first material) is in a range of 0.2 to 10.8.

10. The antimicrobial implant device according to any one of claims 2 to 9, wherein cobalt ions are eluted from the first material in an amount of 7 nmol/l to 81 $\mu$mol/l.

11. The antimicrobial implant device according to any one of claims 1 to 10, wherein
the antimicrobial implant device is an interbody cage;
a body of the interbody cage comprises the second material; and
at least a part of the body of the interbody cage is coated with the first material.

12. The antimicrobial implant device according to claim 11, wherein
at least a part of the body of the interbody cage has a honeycomb structure or a net structure composed of the second material; and
the outer surface of the honeycomb structure or the net structure is coated with the first material.

13. The antimicrobial implant device according to any one of claims 1 to 10, wherein
the antimicrobial implant device is an interbody cage; and
a porous structure composed of intertwined or agglutinated fibers comprising the first material is laminated on a porous structure composed of intertwined or agglutinated fibers comprising the second material.

14. The antimicrobial implant device according to any one of claims 1 to 12, wherein
the antimicrobial implant device is an interbody cage;
a body of the interbody cage comprises a solid portion comprising the second material and a porous section comprising the second material; and
the solid portion and the porous portion are laminated.

15. The antimicrobial implant device according to any one of claims 1 to 12, wherein
the antimicrobial implant device is an interbody cage;
a body of the interbody cage comprises the second material; and
a porous portion comprising the first material is laminated on the porous portion comprising powder particles of the second material.

# Fig. 1

13   14   1

11

12

# Fig. 2

13   14

11

12

# Fig. 3

13   14   11

12

# Fig. 4

# Fig. 5

# Fig. 6

11a

12a

# Fig. 7

11a

12a

# Fig. 8

11b

11c

Fig. 9

11c

11b

Fig. 10

11c

12c

Fig. 11

12c

11c

## INTERNATIONAL SEARCH REPORT

International application No.

PCT/JP2017/007798

| A. CLASSIFICATION OF SUBJECT MATTER |
|---|
| $A61F2/44(2006.01)i$, $A61L27/00(2006.01)i$ |

According to International Patent Classification (IPC) or to both national classification and IPC

| B. FIELDS SEARCHED |
|---|
| Minimum documentation searched (classification system followed by classification symbols)<br>A61F2/44, A61L27/00-27/60 |

Documentation searched other than minimum documentation to the extent that such documents are included in the fields searched

Jitsuyo Shinan Koho      1922-1996    Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | US 2010/0121378 A1 (MALEK, Michel H.),<br>13 May 2010 (13.05.2010),<br>paragraphs [0041], [0044]<br>(Family: none) | 1-15 |
| Y | JP 62-2947 A (Becton, Dickinson and Co.),<br>08 January 1987 (08.01.1987),<br>page 5, upper left column, line 16 to lower<br>right column, line 8; page 8, upper left column,<br>line 10 to lower left column, line 7; fig. 10<br>& US 4886505 A<br>column 3, line 18 to column 4, line 43; column<br>7, lines 15 to 55<br>& BR 8602637 A        & EP 0206024 A2 | 1-15 |

| ☒ Further documents are listed in the continuation of Box C. | ☐ See patent family annex. |
|---|---|

| *   Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>30 March 2017 (30.03.17) | Date of mailing of the international search report<br>11 April 2017 (11.04.17) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2017/007798 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | Hiroaki TAKADAMA et al., "Jinko Kansetsu-yo Titanium Kinzoku eno Seitai Kassei to Kokinsei no Fuyo", Chubu University Annual report of Research Institute of Life and Health Sciences, 2011.03, vol.7, pages 61 to 66 | 3-6,10 |
| X | JP 2013-528411 A  (Deru GmbH), 11 July 2013 (11.07.2013), paragraphs [0022] to [0026]; fig. 1 to 2, 4 to 5 & US 2011/0272276 A1 paragraphs [0034] to [0039] & AU 2011244526 B2        & CA 2796784 A1 & CN 102946842 A         & EP 2382960 A1 & KR 10-2013-0041808 A  & MX 2012012125 A & US 2013/0245783 A1     & WO 2011/131536 A1 | 1-2,9 |
| X | WO 2015/001366 A2  (BAY ZOLTÁN KÖZHASZNÚ NONPROFIT KFT), 08 January 2015 (08.01.2015), page 16, line 5 to page 19, line 26 (Family: none) | 1-2,7-9 |
| A | JP 2002-536048 A  (Synthes AG. Chur), 29 October 2002 (29.10.2002), paragraphs [0007] to [0019]; fig. 1 to 3 & US 2002/0029043 A1 paragraphs [0019] to [0025] & AU 757391 B2          & CA 2360904 A1 & WO 00/45724 A1 | 1-2,7-9 |
| A | JP 2004-526506 A  (MICHELSON, Gary, K), 02 September 2004 (02.09.2004), paragraph [0068] & WO 02/078514 A2 page 21, lines 4 to 29 & AU 2002338239 B2        & CA 2443442 A1 | 11-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5175185 B